# EUROPEAN PATENT SPECIFICATION

(11) **EP 2 439 531 B1**
(45) Date of publication and mention of the grant of the patent: **21.08.2013**
(21) Application number: 11184209.2
(22) Date of filing: 06.10.2011
(51) Int. Cl.: G01N 33/50

(54) **Assay for identifying a malignant, follicular tumour**
Analyse zum Identifizieren eines malignen, follikulären Tumors
Analyse pour identifier une tumeur maligne, folliculaire

(30) Priority: 08.10.2010 GB 201017011
(43) Date of publication of application: 11.04.2012
(73) Proprietor: Skova Services Ltd., Nicosia (CY)
(72) Inventor: Nikolopoulos, Sotiris, Nicosia (CY)
(74) Representative: Elsy, David

(56) References cited:
- KONDO ET AL: "Enhanced B-Raf protein expression is independent of V600E mutant status in thyroid carcinomas", HUMAN PATHOLOGY, SAUNDERS, PHILADELPHIA, PA, US, vol. 38, no. 12, 1 December 2007 (2007-12-01), pages 1810-1818, XP022421077, ISSN: 0046-8177, DOI: 10.1016/J.HUMPATH.2007.04.014
- HOCEVAR M ET AL: "Role of serum thyroglobulin in the pre-operative evaluation of follicular thyroid tumours", 1998, EUROPEAN JOURNAL OF SURGICAL ONCOLOGY 1998 GB, VOL. 24, NR. 6, PAGE(S) 553 - 557, XP002665790, ISSN: 0748-7983 * abstract * * page 554, left-hand column, paragraph 6 - page 555, right-hand column, paragraph 1 *
- LIOU MIAW-JENE ET AL: "Human telomerase reverse transcriptase (hTERT) gene expression in FNA samples from thyroid neoplasms.", 10 March 2003 (2003-03-10), CANCER LETTERS, VOL. 191, NR. 2, PAGE(S) 223-227, XP002665791, ISSN: 0304-3835 * abstract * * page 224, left-hand column, paragraph 3 - page 225, right-hand column, paragraph 1 *
- CASTRO P ET AL: "PAX8-PPAR gamma rearrangement is frequently detected in the follicular variant of papillary thyroid carcinoma", January 2006 (2006-01), JOURNAL OF CLINICAL ENDOCRINOLOGY & METABOLISM, VOL. 91, NR. 1, PAGE(S) 213-220, XP002665792, ISSN: 0021-972X * page 217, left-hand column, paragraph 3 - page 218, left-hand column, paragraph 2 *

## Description

The present invention relates to methods and kits for identifying malignant follicular tumours.

It is accepted that about 10% of the population will develop a clinically significant thyroid nodule during their lifetime. One of the major challenges for the endocrinologists is the evaluation of these thyroid nodules. Although most thyroid nodules are benign, the most common indication for surgical intervention is to exclude the diagnosis of carcinoma. The current criteria for surgery decisions rely on cytopathologic evaluation of cells received from the fine needle aspiration (FNA), a technique that was introduced as a preoperative test for thyroid nodules in the 1970's and validated as a reliable test in numerous studies. Of the malignant FNAs, the majority are from papillary thyroid cancers (PTCs) or its follicular variant (FVPTC). These can be easily diagnosed if they have the classic cytological features, including abundant cellularity and enlarged nuclei containing intranuclear grooves and inclusions. Indeed, one-third of the time these diagnoses are clear on FNA. FNA of thyroid nodules has greatly reduced the need for thyroid surgery and has increased the percentage of malignant tumors among excised nodules. With the adoption of thyroid FNA, the likelihood of requiring surgery for a thyroid nodule decreased about 50%, from 65-70% to 35-40%, with a concomitant decrease in the number of thyroidectomies performed.

Although FNA is currently considered to be the best initial diagnostic test for evaluation of a thyroid nodule, it cannot discriminate between benign and malignant follicular thyroid tumors. Even experienced cytologists encounter problems with FNA samples for which cytological features neither confirm nor rule out malignancy. Patients seen with follicular thyroid lesions are advised to undergo surgery of thyroidectomy. In essence, doctors and patients have to decide preoperatively whether to undergo total thyroidectomy based on inadequate clinical information. Especially the cases designated as suspicious or indeterminate lead to thyroid surgery. Current estimates indicate that carcinomas of the thyroid are ultimately found in 10-20% of lesions read as follicular tumors by FNA cytology. As a consequence, diagnosis of cancer is confirmed by pathological examination of surgical pieces in only about one third of patients operated for cancer or suspicion of cancer, which means that the other two thirds of operated patients could have avoided the surgery.

There is a strong need to develop more accurate initial diagnostic tests for evaluating a thyroid nodule. The advances of molecular biology have been already applied in the field of endocrinology with amazing findings that will certainly aid in the development of better diagnostic test for endocrine pathologies. For example, molecular profiles of follicular adenomas and hyperplastic nodules have been analyzed by oligonucleotide microarray analysis by several groups. At least 400 differentially expressed genes was produced and cluster analysis was able to identify follicular adenomas with a sensitivity of 85% and a specificity of 100%, indicating that benign thyroid lesions can be separated into distinct groups through molecular profiling (Finley et al., 2005). It has also been shown that classic papillary and follicular variant of papillary thyroid tumors can be distinguished from benign thyroid nodules based on their molecular profile with excellent sensitivity and specificity (Huang et al., 2001; Barden et al., 2003; Finlay et al., 2004; Mazzanti et al., 2004; Prasad et al., 2008).

Hocevar and Auersperg, European Journal of Surgical Oncology, 1998, vol. 24, pg. 553-557 and Liou et al., Cancer Letters, 2003, vol. 191, pg. 223-227 analysed molecular markers such as serum thyroglobulin and telomerase reverse transcriptase from FNA samples in order to evaluate thyroid nodules for malignancy.

The inventors, after carefully evaluating the results of these studies, went further and identified a fast and reliable molecular assay based on gene expression analysis which helps to determine accurately the identity of thyroid FNAs and, in particular, suspicious or indeterminate ones. The set of genes that were evaluated include seven genes that have been found to be upregulated in cancerous thyroid tissues, an eighth gene which was found to be upregulated in adenomas of the thyroid tissue and a ninth gene which was utilised for a control housekeeping gene.

The invention provides a method of identifying a malignant follicular tumour comprising:

A method of identifying a malignant follicular tumour, comprising:
(a) determining the level of expression of BRAF-V600E and optionally one or more of the following gene sequences:
   Pax-8 (peroxisome proliferator-activated receptor)
   Adrenomedullin and
   TROP-2
   in a sample from a thyroid nodule and
(b) comparing the level of expression of the genes to a standard.

The method additionally comprises determining the level of expression of one or more genes selected from Galectin 3, MET (HGF receptor) and NRP 2. The best results are obtained by looking at the gene expression of all of the genes. Accordingly, preferably, all of those genes are assayed for expression.

The sample may also be assayed for the level of expression for TFF3. Trefoil Factor 3 was found to be usually upregulated in adenomas of the thyroid tissue, it is usually downregulated in thyroid malignancies.

A further aspect of the invention provides a method of identifying a malignant follicular tumour comprising determining the level of expression of BRAF-V600E and TFF3.

A further aspect provides a method of identifying a malignant follicular tumour comprising determining the level of expression of:
(a) BRAF-V600E
   MET
   Pax-8
   NRP-2 and
   TFF3,
   in a sample from a thyroid nodule and
(b) comparing the level of expression of the genes to a standard.

One or more of Galectin, Adrenomedullin and/or TROP-2 may also be assayed.

The sample from a thyroid nodule is typically a sample of tissues fine needle aspiration (FNA). Increased level of expression of those genes compared to a standard indicates that the sample is likely to comprise malignant follicular tumour tissue.

The standard may, for example, be a predetermined level of expression determined from, for example, the normal level of expression identified from samples obtained from normal, non-malignant thyroid tissue or, alternatively, compared to normal levels obtained from patients with malignant follicular tumour tissue. Some of the genes may be up regulated compared to a standard level in normal tissue. The expression may simply be whether the gene is expressed or not, or whether the gene is expressed in higher levels than non-cancerous tissue. NRP2, MET. Adrenomedullin and Galectin-3 have been observed to be overexpressed in malignant tissue compared to normal tissue.

Preferably, the standard is based upon the level of expression of a control housekeeping gene within the sample from the thyroid nodule. Preferably, that control housekeeping gene is GADPH. However, other housekeeping genes such as the beta-actin gene (ACTB) (Accession# NM_001101.2) and the 60S ribosomal protein L13a gene (RPL13A) (Accession# NM_012423.2).

The level of expression of the or each gene may be determined by measuring the level of mRNA.

Methods of detecting mRNA concentrations are well-known in the art. For example, the level of expression may be determined by real time PCR.

Methods of monitoring hybridisation during PCR to allow target nucleic acid sequences to be quantified are shown, for example, in WO1997/046714 Preferably, the assay utilises double-stranded binding dyes such as SYBR^{™} Green I.

The primer utilised may be two or more of the primers listed below in the materials and methods section.

The method may use an assay kit comprising means to specifically determine the level of expression from the following gene sequences:
BRAF-V600E
Pax-8 (peroxisome proliferator-activated receptor)
Adrenomedullin and
TROP-2

Preferably, the kit comprises means for detecting the level of expression of one of Galectin 3, MET (HGF receptor) and NRP-2. The means may also comprise means for determining the level of all of those genes. Additionally, the kit may comprise means for determining the level of expression of TFF-3 gene. The kit may comprise means for detecting BAAF-600E and two or more of the above genes.

The assay kit may comprise means to specifically determine the level of expression of BRAF-V660E and TFF3.

The assay kit may comprise means to specifically determine the level of expression of:
BRAF-V600E
MET
Pax-8
NRP-2 and
TFF3

A control housekeeping gene, for example, GADPH, may also be tested for by the kits by utilising means for detecting the level of expression of the housekeeping gene. The housekeeping gene may also be ACTB or RPL13A.

The kit is for use in the method of invention and may additionally comprise instructions and/or details of normal levels of expression associated with normal, non-cancerous tissue or cancerous tissue.

The kit may comprise means for detecting the level of expression of a protein obtained from the expression of the or each gene. Such means may, for example, be one or more gene product-specific antibodies or fragments to detect the protein. Such immunogenic assays are generally known in the art using, for example, labelled antibodies. Alternatively, the kit may comprise a pair of primers specific for the or each gene for use in real time PCR.

The invention will now be described by was of example only with reference to the following figures:

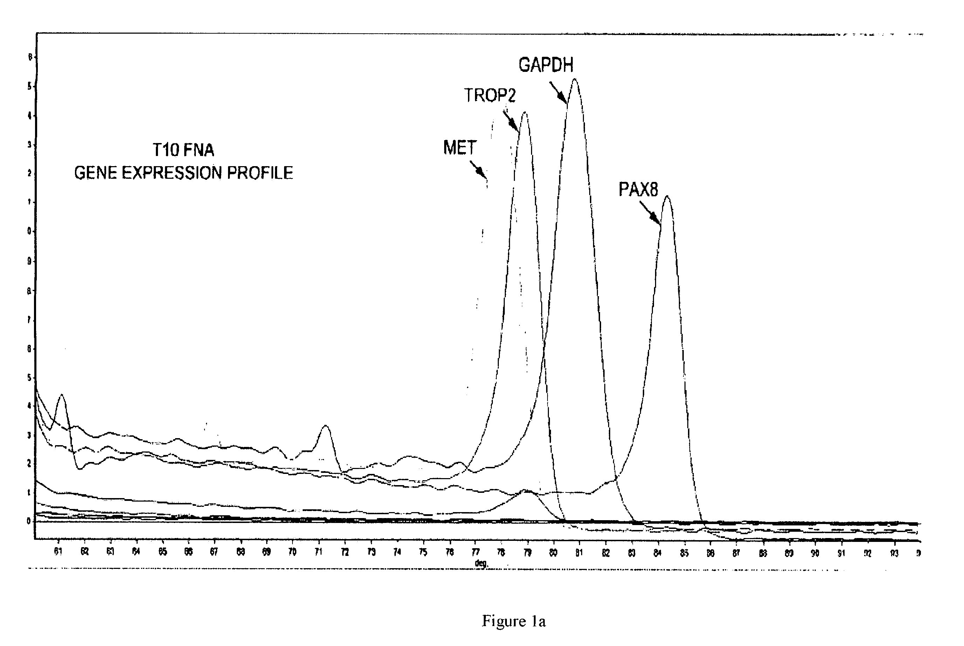

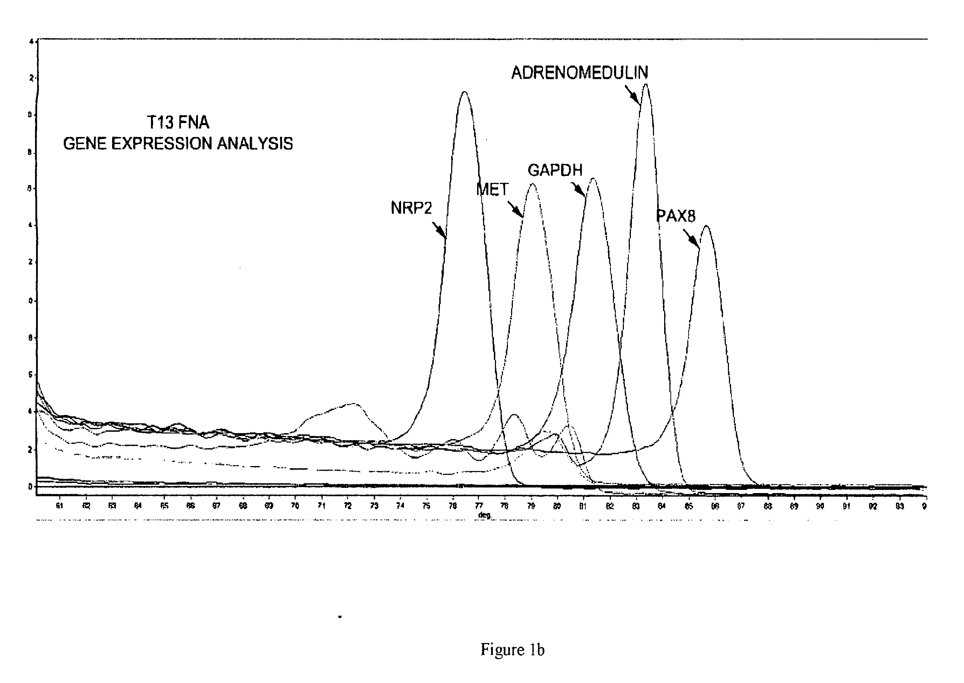

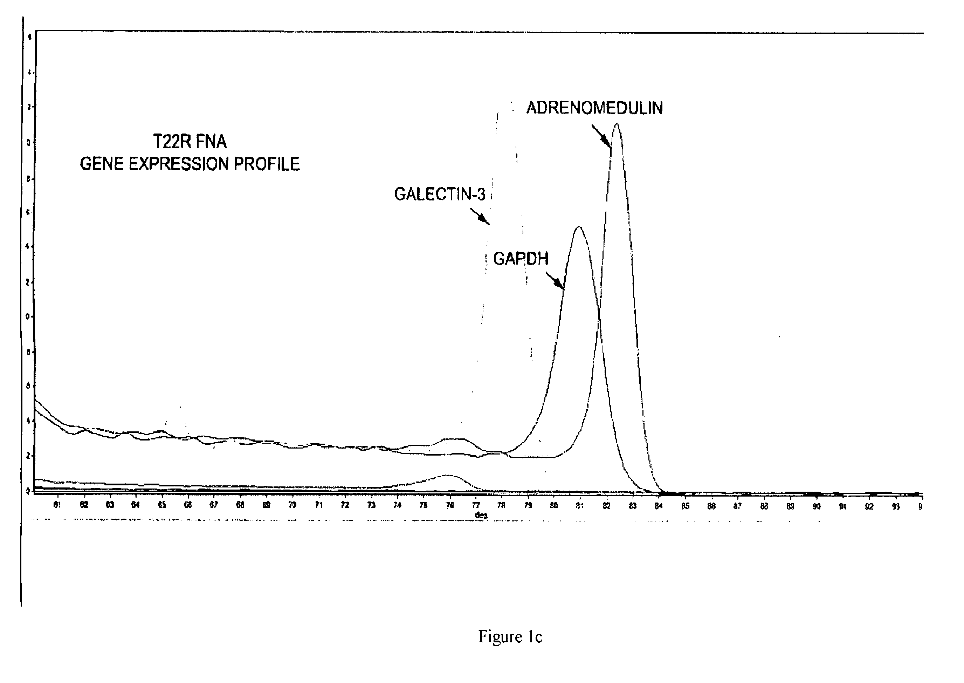

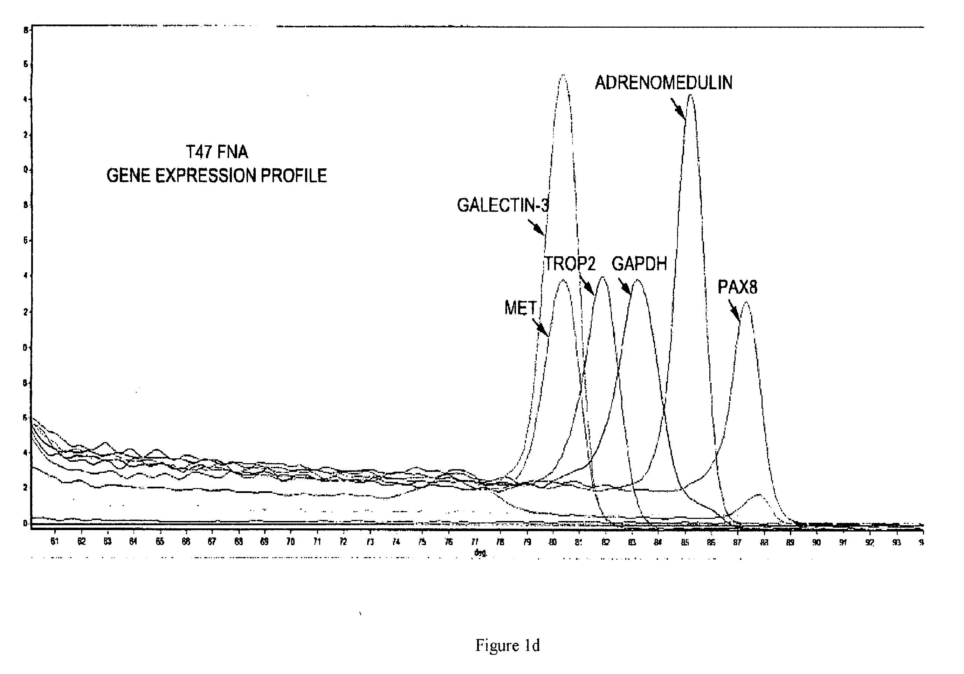

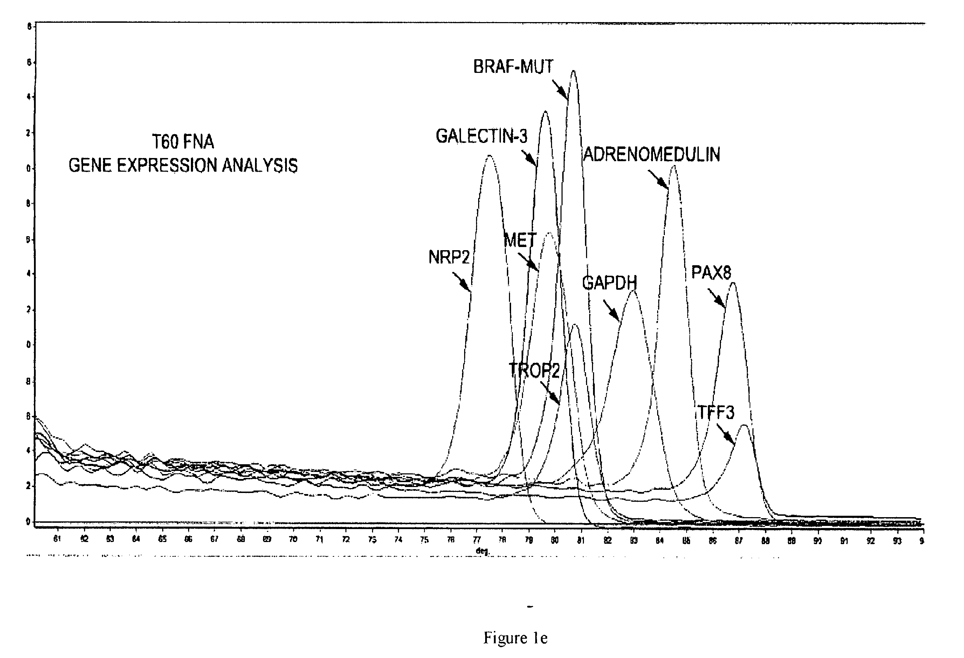

Figure 1 shows gene expression analysis of samples from non-cancerous (a-d) and cancerous (e) FNA samples.

### TECHNICAL DESCRIPTION OF METHODOLOGY

### 1. THYROID FNAs (OBTAINING THE THYROID NODULE SAMPLES)

Thyroid FNAs are performed by an experienced endocrinologist with a thin needle inserted into the thyroid nodule, with subsequent aspiration of cells and/or fluid from the thyroid nodule or mass into the needle. The sample obtained can then be evaluated for the presence of cancerous cells by a cytologist.

For the described methodology a second dip of the needle into thyroid nodule aspirating an equal amount of cells with the first dip is performed and the aspirated material is transferred and washed into an eppendorff tube containing 1ml of a nucleic acids stabilization buffer. The nucleic acid stabilization buffer is prepared as follows.

Stock solutions: 0.5M EDTA disodium dehydrate, 1M Sodium Citrate trisodium salt, dehydrate, Ammonium Sulfate. In 11t beaker we combine 40 ml 0.5M EDTA, 25 ml 1M Sodium Citrate, 700g Ammonium Sulfate and 935 ml nuclease-free water, stirring on a hot plate stirrer on low heat until the ammonium sulfate is completely dissolved. After allowing to cool, adjustment of the pH of the solution to pH 5.2 using 1M H2SO4 and storing at room temperature is performed.

The aspirated FNAs can be stored in the above medium without compromising RNA quality for one week at room temperature, thus allowing easy handling for gene-expression analysis.

### 2. Gene-expression analysis of thyroid FNAs

### STEP 1: RNA isolation

The eppendorff tubes containing the thyroid FNAs aspirates are centrifuged in a microcentrifuge apparatus at 1500rpm for 5 minutes, in order for the collected all cells to the bottom of the tube forming a pellet. The supernatant is removed, leaving behind 10-20 microliters and the resulting pellet is resuspended with 1ml TRIzol-RT reagent (MRC). The isolation of RNA is performed according to the instructions of the manufacturer. A successful FNA sampling for this methodology should yield at least 300ng RNA with excellent quality (A260/280 of 1.6 or higher).

### STEP 2: cDNA sysnthesis

The second step towards the gene-expression analysis of thyroid FNAs is the conversion of the isolated RNA to complimentary DNA (cDNA). The last is performed by the use of the High Capacity cDNA Reverse Transcription Kit (Applied Biosystems), following exactly the instructions of the manufacturer. The final volume of the reaction is 20µl.

### STEP 3: Real-time PCR

The last step of the gene-expression analysis of thyroid FNAs is the real time PCR which is performed using the Platinum® SYBR® Green qPCR SuperMix-UDG (from Invitrogen). This mixture contains SYBR® Green I fluorescent dye, Platinum® *Taq* DNA polymerase, Mg++, uracil-DNA glycosylase (UDG), proprietary stabilizers, and deoxyribonucleotide triphosphates (dNTPs), with dUTP instead of dTTP. The convenient SuperMix formulation delivers excellent sensitivity in the quantification of target sequences, with a linear dose response over a wide range of target concentrations.

SYBR® Green I is a fluorescent dye that binds directly to double-stranded DNA (dsDNA). In qPCR, as dsDNA accumulates, the dye generates a signal that is proportional to the DNA concentration and that can be detected using real-time qPCR instruments. SYBR® Green I in this SuperMix formulation can quantify as few as 10 copies of a target gene in as little as 1 pg of template RNA. Platinum® *Taq* DNA polymerase is precomplexed with specific monoclonal antibodies that inhibit *Taq* DNA polymerase activity during reaction assembly at room temperature. Full polymerase activity is restored after the denaturation step in PCR cycling, providing an automatic hot start PCR. This significantly reduces nonspecific amplification and mispriming and increases amplification efficiency, sensitivity, and yield.

### Template

The target template for real-time PCR is the cDNA generated from the isolated RNA in Step 2 (above) for each FNA biopsy. For the 9 reactions performed for each FNA biopsy, 5 µl (out of 20 µl (~50 ng)) is more than adequate for the required amount of template required to check expression of all target genes.

### Genes analyzed

A brief description of the genes that compose the 9 gene-expression signature follows:
1) ***MET*** a proto-oncogene that encodes a protein MET, also known as **c-Met** or **hepatocyte growth factor receptor (HGFR)**. The proto-oncogene MET product is the hepatocyte growth factor receptor and encodes tyrosine-kinase activity. NCBI reference REFSEQ ID: NM_000Z45
2) ***TROP2*** gene which encodes a transmembrane calcium signal transducer, involved in the regulation of cell- cell adhesion. NCBI reference REFSEQ ID NM_002353
3) ***NRP2*** gene encodes a member of the neuropilin family of receptor proteins. The encoded transmembrane protein interacts with vascular endothelial growth factor (VEGF). NCBI reference REFSEQ ID: NM_201266
4) ***Adrenomedullin*** gene encoding a peptide of 52 amino acids isolated from extracts of human pheochromocytomas arising from adrenal medulla. NCBI reference REFSEQ ID: NM_001124.
5) ***PAX8*** gene, a member of the paired box family of transcription factors. This nuclear protein is involved in thyroid follicular cell development and expression of thyroid-specific genes. NCBI reference REFSEQ ID: NM_003466.
6) ***Galectin-3* gene**, **LGALS3**, is a member of the lectin family. It is expressed in the nucleus, cytoplasm, mitochondria, cell surface, and extracellular space. This protein has been shown to be involved in the following biological processes: cell adhesion, cell activation and chemoattraction, cell growth and differentiation, cell cycle, and apoptosis. NCBI reference REFSEQ ID: NM_002306.
7) ***Trefoil factor 3** (TFF3)* gene is a member of the trefoil family. They are stable secretory proteins expressed in gastrointestinal mucosa. TFF3 is thought to be involved in protection from injury and regenerative growth and repair of intestinal epithelial cells. NCBI reference REFSEQ ID: NM_003226.
8) **B-Raf proto-oncogene serine/threonine-protein kinase** *(B-RAF).* The B-RAF protein is involved in sending signals in cells and in cell growth. The *BRAF* gene becomes mutated in thyroid cancer and this mutated type of *BRAF(v600E)* is typically assayed in the described test. NCBI reference REFSEQ ID: NM_003226.
9) **Glyceraldehyde-3-phosphate dehydrogenase (GAPDH)** gene is one of the most commonly used housekeeping genes used in comparisons of gene expression data. The GAPDH protein is an enzyme that catalyzes the sixth step of glycolysis and thus serves to break down glucose for energy and carbon molecules. NCBI reference REFSEQ ID: NM_002046.

### Primers

The primer sequences that we designed and used for the detection of all analyzed genes is listed below.

| **Sequence Name** | **Sequence** |
|---|---|
| MET-FWD | TTC TGA CCG AGG GAA TCA |
| MET-REV | CCT TCA CTT CGC AGG CAG |
| BRAF V600E-WT-FWD | GTG ATT TTG GTC TAG CTA |
| BRAF V600E-MUT- | GTG ATT TTG GTC TAG CTA |
| PAX-8 FWD | TGC CTC ACA ACT CCA TCA |
| PAX-8 REV | CAG GTC TAC GAT GCG CTG |
| GALECTIN-3-FWD | CTG GGG AAG GGA AGA AAG |
| GALECTIN-3-REV | GAG CAT CAT TCA CTG CAA |
| ADRENOMEDULIN- | ACG GAA ACC AGC TTC ATC C |
| ADRENOMEDULIN- | GCT TGG ACT TCG GAG TTT |
| TROP2-FWD | AAT GTA TCC CCT TIC GGT |
| TROP2-REV | TCC CGG GTT GTC ATA CAG |
| NRP2-FWD | GGC TTT TGC AGG TGA GAA |
| NRP2-REV | TGC TCC AGT CCA CCT CGT |
| GAPDH-FWD | AAG GTG AAG GTC GGA GTC |
| GAPDH-REV | AAT GAA GGG GTC ATT GAT |
| TFF3-FWD | CTC CAG CTC TGC TGA GGA |
| TFF3-REV | CAC TCC TTG GGG GTG ACA |

### Reaction conditions for the real-time PCR reaction

### Cycling Program

50°C for 2 min hold
95°C for 5 min hold 45
cycles of: 95°C, 15 s
   58°C, 20s
   72°C, 30s

After the end of the Cycling Program, a Melting Curve Analysis Program is performed in order to evaluate and confirm the correct real-time PCR products from their melting temperatures:

### Melting Curve Analysis Program

Ramp from 60 to 94
Rising by 0.2 degrees each step
Wait for 90 seconds
Wait for 1 second for each step afterwards

### STEP 4: Evaluation of Real-time PCR results

All cancerous samples analyzed to date were found to highly express all genes listed above with the exception of TFF3 that is usually expressed by cancer cells at low levels, if not expressed at all. Non-cancerous samples were found not to express the mutated gene BRAF V600E as well as at least two or more genes from the above list. In addition, adenomas usually express high levels of TFF3. TFF3 was found to be down regulated in thyroid malignancies.

Most tumours had BRAF-V600E, MET, Pax-8, NRP-2 and TFF3 expressed. Adrenomedullin and TROP2 were also expressed in some tumours.

Figure 1 shows examples of expression profiles of examples of different samples. T10, T13, T22R and T47 were not assessed to be cancerous. T60 (Figure 1e) was expressed to be cancerous. The presence of the BRAF-V600E mutation ("BRAF-MUT") and typically TFF3 was shown to be key in allowing the discrimination between cancerous and non-cancerous.

Similar patterns of the presence of BRAF-V600E and typically TFF3 were seen in other cancerous tissue samples.

### CONCLUSION

Molecular classification of tissues is an emerging technology that will undoubtedly change the way patients are managed in the future. The described methodology uses this technology in order to provide a reliable method for an accurate molecular diagnosis of thyroid nodules. The claimed patent identifies a gene signature that enhances the sensitivity and the specificity of diagnosis of thyroid nodules when compared to traditional cytological techniques. Current diagnosis is conventionally obtained by morphological examination of tissue biopsy sections but is not totally reliable due to the nature of the thyroid tissue. Nevertheless, intra- and inter-observer incongruities may occur. The RT-qPCR gene profiling method based on the gene signature described here is an accurate tool for molecular diagnosis of certain pathologies of thyroid such as the presence/absence of cancerous or pre-cancerous cells not subjected to intra- and inter-observer error. The method would enhance diagnosis, particularly in those cases in which it is necessary to resolve uncertainties.

### Bibliography

Adeniran AJ, Zhu Z, Gandhi M, Steward DL, Fidler JP, Giordano TJ, Biddinger PW, Nikiforov YE 2006. Correlation between genetic alterations and microscopic features, clinical manifestations, and prognostic characteristics of thyroid papillary carcinomas. Am J Surg Pathol 30:216-222
Finley DJ, Arora N, Zhu B, Gallagher L, Fahey TJ 3rd. 2004 Molecular profiling distinguishes papillary carcinoma from benign thyroid nodules. J Clin Endocrinol Metab. 2004 Jul;89(7):3214-23.
Finley DJ, Lubitz CC, Wei C, Zhu B, Fahey TJ 3rd. Advancing the molecular diagnosis of thyroid nodules: defining benign lesions by molecular profiling. Thyroid. 2005 Jun;15(6):562-8.
Griffith OL, Melck A, Jones SJ, Wiseman SM. Meta analysis and meta-review of thyroid cancer gene expression profiling studies identifies important diagnostic biomarkers. J Clin Oncol 2006;24:5043-51.
Hoffmann S, Maschuw K, Hassan I, Reckzeh B, Wunderlich A, Lingelbach S, Zielke A. Differential pattern of integrin receptor expression in differentiated and anaplastic thyroid cancer cell lines. Thyroid. 2005 Sep; 15(9): 1011-20.
Huang, Y., Prasad, M., Lemon, W. J., Hampel, H., Wright, F. A., Kornacker, K., LiVolsi, V., Frankel, W., Kloos, R. T., Eng, C., Pellegata, N. S., and de la Chapelle, A. Gene expression in papillary thyroid carcinoma reveals highly consistent profiles. Proc. Natl. Acad. Sci. USA, 98: 15044-15049, 2001.
Illario M, Amideo V, Casamassima A, Andreucci M, di Matola T, Miele C, Rossi G, Fenzi G, Vitale M. Integrin-dependent cell growth and survival are mediated by different signals in thyroid cells. J Clin Endocrinol Metab. 2003 Jan;88(1):260-9.
Kimura ET, Nikiforova MN, Zhu Z, Knauf JA, Nikiforov YE, Fagin JA 2003. High prevalence of BRAF mutations in thyroid cancer: genetic evidence for constitutive activation of the RET/PTC-RAS-BRAF signaling pathway in papillary thyroid carcinoma. Cancer Res 63:1454-1457
Mazzanti C, Zeiger MA, Costouros NG, Umbricht C, Westra WH, Smith D, Somervell H, Bevilacqua G, Alexander HR, Libutti SK. 2004. Using gene expression profiling to differentiate benign versus malignant thyroid tumors. Cancer Res. 2004 Apr 15;64(8):2898-903.
Nikiforova MN, Lynch RA, Biddinger PW, Alexander EK, Dorn 2nd GW, Tallini G, Kroll TG, Nikiforov YE 2003. RAS point mutations and PAX8-PPAR rearrangement in thyroid tumors: evidence for distinct molecular pathways in thyroid follicular carcinoma. J Clin Endocrinol Metab 88:2318-2326
Pizzolanti G, Russo L, Richiusa P, Bronte V, Nuara RB, Rodolico V, Amato MC, Smeraldi L, Sisto PS, Nucera M, Bommarito A, Citarrella R, Lo Coco R, Cabibi D, Lo Coco A, Frasca F, Gulotta G, Latteri MA, Modica G, Galluzzo A, Giordano C 2007. Fine-needle aspiration molecular analysis for the diagnosis of papillary thyroid carcinoma through BRAF V600E mutation and RET/PTC rearrangement. Thyroid 17:1109-1115
Prasad NB, Somervell H, Tufano RP, Dackiw AP, Marohn MR, Califano JA, Wang Y, Westra WH, Clark DP, Umbricht CB, Libutti SK, Zeiger MA. 2008. Identification of genes differentially expressed in benign versus malignant thyroid tumors. Clin Cancer Res. 2008 Jun 1;14(11):3327-37.
Salvatore G, Giannini R, Faviana P, Caleo A, Migliaccio I, Fagin JA, Nikiforov YE, Troncone G, Palombini L, Basolo F, Santoro M 2004. Analysis of BRAF point mutation and RET/PTC rearrangement refines the fine-needle aspiration diagnosis of papillary thyroid carcinoma. J Clin Endocrinol Metab 89:5175- 5180
Sapio MR, Guerra A, Posca D, Limone PP, Deandrea M, Motta M, Troncone G, Caleo A, Vallefuoco P, Rossi G, Fenzi G, VitaleM 2007. Combined analysis of galectin-3 and BRAFV600E improves the accuracy of fine-needle aspiration biopsy with cytological findings suspicious for papillary thyroid carcinoma. Endocr Relat Cancer 14:1089-1097
Trusolino L, Bertotti A, Comoglio PM. A signaling adapter function for alpha6beta4 integrin in the control of HGF-dependent invasive growth. Cell. 2001 Nov 30;107(5):643-54.
Xing M, Tufano RP, Tufaro AP, Basaria S, Ewertz M, Rosenbaum E, Byrne PJ, Wang J, Sidransky D, Ladenson PW 2004. Detection of BRAF mutation on fine needle aspiration biopsy specimens: a new diagnostic tool for papillary thyroid cancer. J Clin Endocrinol Metab 89:2867-2872

## Claims

1. A method of identifying a malignant follicular tumour, comprising:
(a) determining the level of expression of BRAF-V600E and optionally one or more of the following gene sequences:
Pax-8 (peroxisome proliferator-activated receptor)
Adrenomedullin and
TROP-2
in a sample from a thyroid nodule and
(b) comparing the level of expression of the genes to a standard.

2. A method according to any preceding claim, wherein the level of expression of the or each gene is determined by measuring the level of mRNA from the or each gene in the sample.

3. A method according to any preceding claim, additionally comprising detecting the level of expression of the TFF3 gene in the sample.

4. A method according to claim 1 to 3, additionally comprising determining the level of expression of one or more genes selected from Galectin-3, MET (HGF receptor) and NRP-2.

5. A method according to any preceding claim, wherein BRAF-V600E and two or more of Pax-8, adrenomedullin, TROP-2, galectin-3, MET, NRP-2 or TFF-3, or preferably the expression of all of said gene sequences, in the sample is determined.

6. A method of identifying a malignant follicular tumour according to claim 4 comprising determining the level of expression from the following sequences:
BRAF-V600E
MET
Pax-8
NRP-2 and
TFF3
in a sample from a thyroid nodule and comparing the level of expression of the genes to a standard.

7. A method according to any preceding claim, wherein the level of expression of a control housekeeping gene is determined in the sample.

8. A method according to claim 7, wherein the control housekeeping gene is GADPH.

9. A method according to any preceding claim, wherein the level of expression of each gene is determined by real time PCR.

## Patentansprüche

1. Verfahren zum Identifizieren eines malignen follikulären Tumors, welches umfasst:
(a) Bestimmen des Expressionsniveaus von BRAF-V600E und gegebenenfalls eines oder mehrerer der folgenden Gensequenzen:
Pax-8 (Peroxisomenproliferator-aktivierter Rezeptor)
Adrenomedullin und
TROP-2
in einer Probe aus einem Schilddrüsenknoten und
(b) Vergleichen des Expressionsniveaus der Gene in Bezug auf einen Standard.

2. Verfahren nach irgendeinem der vorhergehenden Ansprüche, wobei das Expressionsniveau des jeweiligen Gens durch Messen der Niveaus von mRNA aus dem jeweiligen Gen in der Probe bestimmt wird.

3. Verfahren nach irgendeinem der vorhergehenden Ansprüche, welches zusätzlich das Erfassen des Expressionsniveaus des TFF3-Gens in der Probe umfasst.

4. Verfahren nach einem der Ansprüche 1 bis 3, welches zusätzlich das Bestimmen des Expressionsniveaus eines oder mehrerer Gene umfasst, welche ausgewählt sind aus: Galectin-3, MET (HGF-Rezeptor) und NRP-2.

5. Verfahren nach irgendeinem der vorhergehenden Ansprüche, wobei BRAF-V600E und zwei oder mehrere der Pax-8, Adrenomedullin, TROP-2, Galectin-3, MET, NRP-2 oder TFF-3 oder vorzugsweise die Expression sämtlicher der genannten Gensequenzen in der Probe bestimmt werden.

6. Verfahren zum Identifizieren eines malignen follikulären Tumors gemäß Anspruch 4, welches das Bestimmen der Expressionsniveaus aus folgenden Sequenzen umfasst:
BRAF-V600E
MET
Pax-8
NRP-2 und
TFF3
in einer Probe aus einem Schilddrüsenknoten und Vergleichen der Expressionsniveaus der Gene in Bezug auf einen Standard.

7. Verfahren nach irgendeinem der vorhergehenden Ansprüche, wobei das Expressionsniveau eines Kontroll-Hauskeeper-Gens in der Probe bestimmt wird.

8. Verfahren nach Anspruch 7, wobei das Kontroll-Hauskeeper-Gen GADPH ist.

9. Verfahren nach irgendeinem der vorhergehenden Ansprüche, wobei das Expressionsniveau jedes Gens durch Echtzeit-PCR bestimmt wird.

## Revendications

1. Procédé d'identification d'une tumeur folliculaire maligne, comprenant :
(a) la détermination du niveau d'expression de BRAF-V600E et éventuellement d'une ou plusieurs des séquences géniques suivantes :
Pax-8 (récepteur activé par les proliférateurs de peroxysomes)
adrénomédulline et
TROP-2
dans un échantillon provenant d'un nodule thyroïdien et
(b) la comparaison du niveau d'expression des gènes à un étalon.

2. Procédé selon l'une quelconque des revendications précédentes où le niveau d'expression du ou de chaque gène est déterminé en mesurant le niveau d'ARNm provenant du ou de chaque gène dans l'échantillon.

3. Procédé selon l'une quelconque des revendications précédentes comprenant en outre la détection du niveau d'expression du gène TFF3 dans l'échantillon.

4. Procédé selon les revendications 1 à 3 comprenant en outre la détermination du niveau d'expression d'un ou plusieurs gènes choisis parmi Galectine-3, MET (récepteur de HGF) et NRP-2.

5. Procédé selon l'une quelconque des revendications précédentes où BRAF-V600E et deux ou plusieurs de Pax-8, adrénomédulline, TROP-2, galectine-3, MET, NRP-2 ou TFF-3, ou de préférence l'expression de toutes lesdites séquences géniques, dans l'échantillon est déterminée.

6. Procédé d'identification d'une tumeur folliculaire maligne selon la revendication 4 comprenant la détermination du niveau d'expression provenant des séquences suivantes :
BRAF-V600E
MET
Pax-8
NRP-2 et
TFF3
dans un échantillon provenant d'un nodule thyroïdien et la comparaison du niveau d'expression des gènes à un étalon.

7. Procédé selon l'une quelconque des revendications précédentes où le niveau d'expression d'un gène domestique témoin est déterminé dans l'échantillon.

8. Procédé selon la revendication 7 où le gène domestique témoin est GADPH.

9. Procédé selon l'une quelconque des revendications précédentes où le niveau d'expression de chaque gène est déterminé par PCR en temps réel.


<110> Skova Services Limited
<120> Assay
<130> p336462ep/de
<150> gb1017011.6
   <151> 2010-10-08
<160> 18
<170> PatentIn version 3.5
<210> 1
   <211> 18
   <212> DNA
   <213> Homo sapiens
<400> 1
   ttctgaccga gggaatca 18
<210> 2
   <211> 18
   <212> DNA
   <213> Homo sapiens
<400> 2
   ccttcacttc gcaggcag 18
<210> 3
   <211> 18
   <212> DNA
   <213> Homo sapiens
<400> 3
   gtgattttgg tctagcta 18
<210> 4
   <211> 18
   <212> DNA
   <213> Homo sapiens
<400> 4
   gtgattttgg tctagcta 18
<210> 5
   <211> 18
   <212> DNA
   <213> Homo sapiens
<400> 5
   tgcctcacaa ctccatca 18
<210> 6
   <211> 18
   <212> DNA
   <213> Homo sapiens
<400> 6
   caggtctacg atgcgctg 18
<210> 7
   <211> 18
   <212> DNA
   <213> Homo sapiens
<400> 7
   ctggggaagg gaagaaag 18
<210> 8
   <211> 18
   <212> DNA
   <213> Homo sapiens
<400> 8
   gagcatcatt cactgcaa 18
<210> 9
   <211> 19
   <212> DNA
   <213> Homo sapiens
<400> 9
   acggaaacca gcttcatcc 19
<210> 10
   <211> 18
   <212> DNA
   <213> Homo sapiens
<400> 10
   gcttggactt cggagttt 18
<210> 11
   <211> 18
   <212> DNA
   <213> Homo sapiens
<400> 11
   aatgtatccc ctttcggt 18
<210> 12
   <211> 18
   <212> DNA
   <213> Homo sapiens
   400> 12
<210> 13
   <211> 18
   <212> DNA
   <213> Homo sapiens
<400> 13
   ggcttttgca ggtgagaa 18
<210> 14
   <211> 18
   <212> DNA
   <213> Homo sapiens
<400> 14
   tgctccagtc cacctcgt 18
<210> 15
   <211> 18
   <212> DNA
   <213> Homo sapiens
<400> 15
   aaggtgaagg tcggagtc 18
<210> 16
   <211> 18
   <212> DNA
   <213> Homo sapiens
<400> 16
   aatgaagggg tcattgat 18
<210> 17
   <211> 18
   <212> DNA
   <213> Homo sapiens
<400> 17
   ctccagctct gctgagga 18
<210> 18
   <211> 18
   <212> DNA
   <213> Homo sapiens
<400> 18
   cactccttgg gggtgaca 18